# FASCICULE DE BREVET EUROPEEN

(11) **EP 4 410 231 B1**
(45) Date de publication et mention de la délivrance du brevet: **20.08.2025**
(21) Numéro de dépôt: 23154772.0
(22) Date de dépôt: 02.02.2023
(51) Int. Cl.: A61B 90/30

(54) **DISPOSITIF D' ÉCLAIRAGE**
BELEUCHTUNGSVORRICHTUNG
LIGHTING DEVICE

(43) Date de publication de la demande: 07.08.2024
(73) Titulaire: Steris, 33185 Le Haillan (FR)
(72) Inventeur: CHOROSY, Damien, 33800 BORDEAUX (FR)
(74) Mandataire: Ernest Gutmann - Yves Plasseraud S.A.S.

(56) Documents cités:
- EP-A1- 3 056 164
- EP-A1- 3 081 857
- US-A1- 2016 302 879
- US-A1- 2021 239 299

## Description

### Domaine de la divulgation

L'invention concerne un dispositif d'éclairage médical, notamment pour un bloc opératoire.

### Etat de la technique

Un tel dispositif d'éclairage comporte classiquement au moins un bras articulé dont une extrémité est fixe et dont une extrémité opposée mobile est équipée d'un module d'éclairage.

Le module d'éclairage peut être déplacé par un chirurgien, de manière à orienter le faisceau lumineux vers une zone à éclairer d'un patient.

Un tel dispositif est notamment connu du document EP 3 081 854.

Celui-ci comporte classiquement un corps logeant des modules d'éclairage, monté sur au moins un bras articulé, et une poignée solidaire du corps. Ladite poignée permet de déplacer manuellement le corps en tout point de l'espace et d'orienter le corps et les modules d'éclairage dans toutes les directions. La poignée est classiquement fixée par vissage sur le corps.

L'invention vise à proposer une alternative à une telle fixation de la poignée sur le corps.

### Exposé de la divulgation

A cet effet, le présent document propose un dispositif d'éclairage comportant un corps équipé d'au moins un module d'éclairage et d'une poignée montée sur le corps, caractérisé en ce que le corps comporte au moins un plot d'accrochage et des moyens d'indexation, la poignée comportant au moins une embase apte à pivoter autour d'un axe par rapport au corps entre une première position angulaire formant une position déverrouillée de l'embase par rapport au corps et une seconde position angulaire formant une position verrouillée de l'embase sur le corps, ladite embase comportant au moins un trou oblong s'étendant circonférentiellement autour de l'axe et apte à coopérer avec le plot d'accrochage de façon à :
- autoriser le déplacement circonférentiel du plot d'accrochage le long du trou oblong correspondant entre les positions verrouillée et déverrouillée de l'embase par rapport au corps,
- autoriser la translation selon ledit axe de l'embase par rapport au corps dans la position déverrouillée de l'embase par rapport au corps, et
- interdire ladite translation dans la position verrouillée de l'embase par rapport au corps,
les moyens d'indexation du corps étant aptes à coopérer avec des moyens d'indexation complémentaires de l'embase, de façon à :
- autoriser la rotation de l'embase par rapport au corps, de la position déverrouillée vers la position verrouillée,
- interdire la rotation de l'embase par rapport au corps, une fois la position déverrouillée atteinte.

Les termes axial, radial et circonférentiel sont définis par rapport à l'axe de rotation de l'embase.

Le plot et le trou oblong forment ainsi des moyens de verrouillage de type baïonnette, où la rotation de l'embase par rapport au corps permet de verrouiller aisément l'embase par rapport au corps. De tels moyens de fixation ont une structure relativement simple et permettent un montage aisé de l'embase, tout en assurant une fixation sécurisée de la poignée sur le corps.

Le plot d'accrochage peut s'étendre parallèlement à l'axe de rotation de l'embase par rapport au corps.

Le plot d'accrochage peut comporter une partie principale et extrémité libre de plus grande dimension que la partie principale, le trou oblong de la base comporte une première partie angulaire de dimension supérieure à l'extrémité libre et une seconde partie angulaire de dimension inférieure à l'extrémité libre, la partie principale du plot s'étendant dans la première partie angulaire du trou oblong dans la position déverrouillée, la partie principale du plot s'étendant dans la seconde partie angulaire du trou oblong dans la position verrouillée.

Ainsi, dans la position déverrouillée, l'extrémité libre ou tête du plot peut traverser le trou oblong et autoriser la mise en place ou le retrait de l'embase par rapport au corps, par translation de ladite embase. Par ailleurs, dans la position verrouillée, la tête du plot vient en butée sur les bords de la seconde partie angulaire du trou oblong, de manière à empêcher le déplacement en translation, et donc le retrait, de l'embase par rapport au corps.

La partie principale du plot d'accrochage peut être cylindrique et présenter un premier diamètre.

L'extrémité libre du plot d'accrochage peut être cylindrique et présenter un second diamètre, supérieur au premier diamètre.

On définit par largeur du trou oblong la dimension du trou oblong perpendiculairement à la direction d'extension dudit trou oblong, c'est-à-dire la dimension du trou oblong selon la direction radiale.

La largeur de la première partie du trou oblong peut être supérieure au second diamètre. La largeur de la seconde partie du trou oblong peut être inférieure au second diamètre et supérieure au premier diamètre.

Le corps peut comporter au moins trois plots d'accrochage répartis autour de l'axe de rotation de l'embase, coopérant respectivement avec trois trous oblongs de l'embase.

Le nombre de plots d'accrochage et de trous oblongs peut être égal à trois.

Les plots d'accrochage et les trous oblongs peuvent être répartis de façon non régulière autour de l'axe de rotation de l'embase.

Une telle caractéristique permet d'assurer une fonction de détrompage, en particulier si l'embase comporte des éléments annexes devant être positionnés correctement par rapport à la base.

Les éléments annexes peuvent être des moyens de connexion électrique, par exemple.

Les moyens d'indexation du corps, ou respectivement les moyens d'indexation complémentaires de l'embase, peuvent comporter un poussoir comprenant une partie mobile entre une position activée et une position désactivée, ladite partie mobile étant soumise à l'action d'un élément de rappel élastique tendant à rappeler ladite partie mobile du poussoir vers sa position activée, les moyens d'indexation complémentaires de l'embase, ou respectivement les moyens d'indexation du corps, comportant un logement, ladite partie mobile du poussoir étant apte à être logée en partie dans ledit logement de l'embase en position activée.

La partie mobile peut être un doigt d'indexage.

La partie mobile peut être une bille.

L'élément de rappel élastique peut être un ressort, par exemple un ressort de compression. Le ressort peut être un ressort hélicoïdal.

Le ressort et le doigt d'indexage peuvent être logés, au moins en partie, dans un corps du poussoir. Le poussoir peut être vissé dans le corps.

Le corps et/ou l'embase peut être équipée de moyens de désactivation des moyens d'indexation, aptes à libérer en rotation l'embase par rapport au corps.

Lorsque l'embase est déplacée en rotation vers la position verrouillée, elle peut être maintenue en rotation par activation des moyens d'indexation.

Afin de pouvoir démonter l'embase du corps, il est possible de désactiver les moyens d'indexation, puis de déplacer l'embase vers sa position déverrouillée.

Les moyens de désactivation peuvent comporter un bouton poussoir apte à repousser la partie mobile du poussoir vers sa position désactivée, à l'encontre de l'effort exercé par l'élément de rappel élastique.

Le bouton poussoir peut être logé dans un logement. Ledit logement peut déboucher en regard des moyens d'indexation.

L'embase peut être apte à pivoter, autour de l'axe et entre les deux positions angulaires verrouillée et déverrouillée, d'un angle compris entre 5 et 25°, par exemple égal à 15°.

La poignée peut comporter une partie amovible, fixée de façon amovible sur l'embase par l'intermédiaire de moyens de verrouillage.

La partie amovible peut ainsi être retirée de façon à faciliter le remplacement et/ou la stérilisation de ladite poignée.

Le dispositif d'éclairage peut être un dispositif d'éclairage médical. Le dispositif d'éclairage peut être une lampe scialytique, notamment pour un bloc opératoire.

Le terme "bloc opératoire" inclut également les cas où le dispositif d'éclairage est adapté à un fauteuil dentaire ou à un domaine d'activité similaire : salles d'examen, salles d'urgence, salles de plâtre, unités de soins intensifs, chambres de suture, chambres à induction, néonatologie, pédiatrie, maternité,....

Le dispositif peut comporter une embase, par exemple une embase mobile équipée de roues, à partir de laquelle s'étend un mat, un bras étant articulé sur le mat, par exemple à l'extrémité supérieure du mat. Une tête ou coupole d'éclairage comportant le corps peut être montée sur l'extrémité libre du bras. Le bras articulé peut comporter plusieurs éléments reliés les uns aux autres par des articulations permettant de déplacer manuellement la tête d'éclairage en tout point de l'espace et d'orienter cette tête dans toutes les directions.

En variante, le bras articulé peut être relié à un point fixe d'un mur ou d'un plafond, par exemple, et non à un mat solidaire d'une embase.

Plusieurs modules d'éclairage peuvent être montés à l'intérieur du corps. Au moins un module d'éclairage peut comporter un bloc optique et = une carte électronique sur laquelle sont montées des sources lumineuses, à savoir des diodes électroluminescentes, agencées en regard de collimateurs du bloc optique.

Les cartes électroniques des modules peuvent permettre de gérer l'allumage des différentes sources lumineuses.

Le dispositif peut également comporter des moyens de commande aptes notamment à commander l'allumage des différents modules et peut comprendre à cet effet une carte électronique de commande. Ladite carte électronique de commande peut être montée et fixée dans le corps.

Un capot transparent peut refermer le corps, en recouvrant notamment les modules d'éclairage.

Les documents US 2021/239299 A1 et EP 3 081 857 A1 divulguent des dispositifs d'éclairage de l'art antérieur.

### Brève description des figures

D'autres caractéristiques et avantages de la présente divulgation apparaîtront dans la description détaillée suivante, se référant aux dessins annexés dans lesquels :
[Fig. 1] est une vue en perspective d'un dispositif d'éclairage selon une forme de réalisation du présent document,
[Fig. 2] est une vue éclatée, en perspective, d'une partie du dispositif de la figure 1,
[Fig. 3] est une vue en perspective d'un moyeu du corps du dispositif,
[Fig. 4] est une vue en perspective, de l'avant, de la poignée,
[Fig. 5] est une vue en perspective, de l'arrière, de la poignée,
[Fig. 6] est une vue arrière de la poignée,
[Fig. 7] est une vue en coupe axiale de la poignée,
[Fig. 8] est une vue en coupe axiale de la poignée et du moyeu.

### Description détaillée de la divulgation

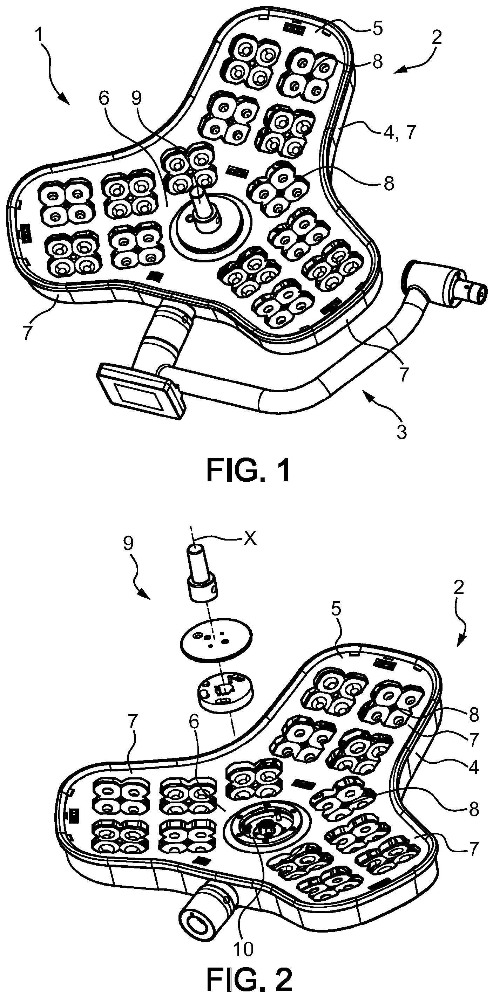

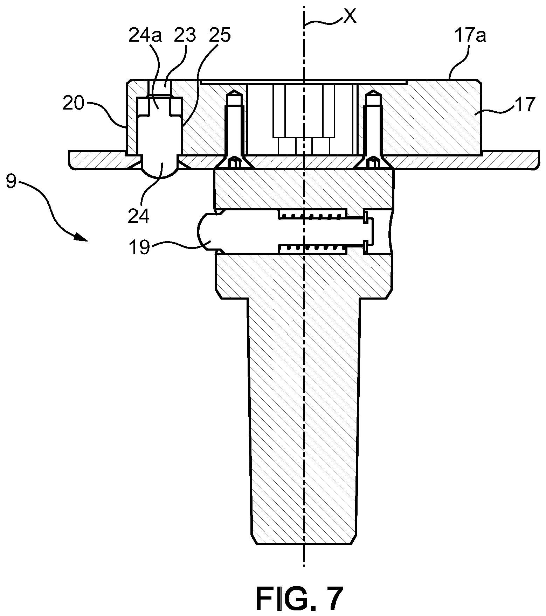

Les figures 1 à 8 représentent un dispositif d'éclairage 1 pour bloc opératoire, selon une forme de réalisation du présent document.

Celui-ci comporte une tête ou coupole d'éclairage 2 et est montée sur l'extrémité libre d'un bras articulé 3.

La tête d'éclairage 2 comporte un corps creux 4 dont l'ouverture est recouverte d'un capot ou écran transparent de protection 5.

Le corps 4 comporte une partie centrale 6 à partir de laquelle s'étendent trois lobes 7. Des modules d'éclairage 8 sont montés à l'intérieur du corps 4. Chaque module 8 comporte un bloc optique et une carte électronique sur laquelle sont montées quatre sources lumineuses, à savoir des diodes électroluminescentes, agencées en regard de collimateurs formés par le bloc optique.

Le dispositif 1 comporte également des moyens de commande aptes notamment à commander l'allumage des différents modules.

Une poignée 9 est montée sur le corps 4, en particulier sur un moyeu 10 du corps 4.

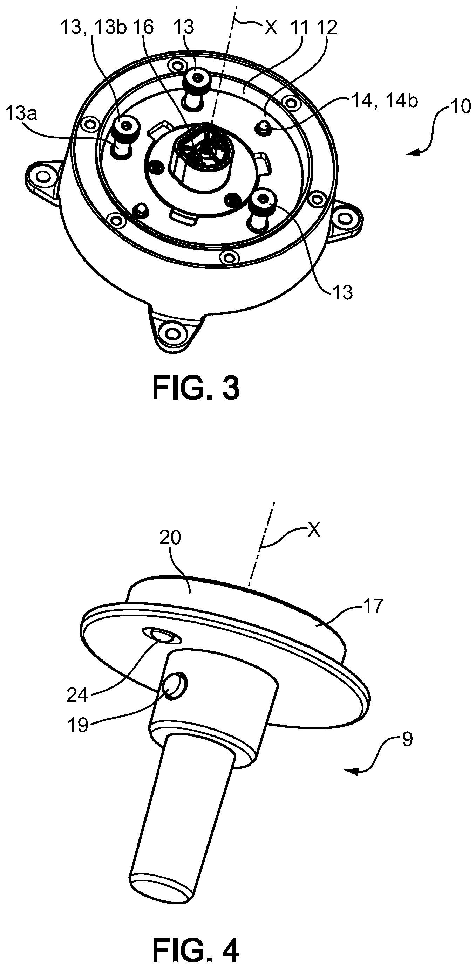

Le moyeu 10 présente par exemple une forme circulaire d'axe X et délimite une surface de guidage cylindrique 11 et une surface de fond 12. Trois plots d'accrochage 13 (figure 3) s'étendant parallèlement à l'axe X et sont répartis de façon non régulière autour de l'axe X.

Chaque plot d'accrochage 13 comporte une partie principale 13a cylindrique présentant un premier diamètre et une extrémité libre ou tête 13b, présentant un second diamètre, supérieur au premier diamètre.

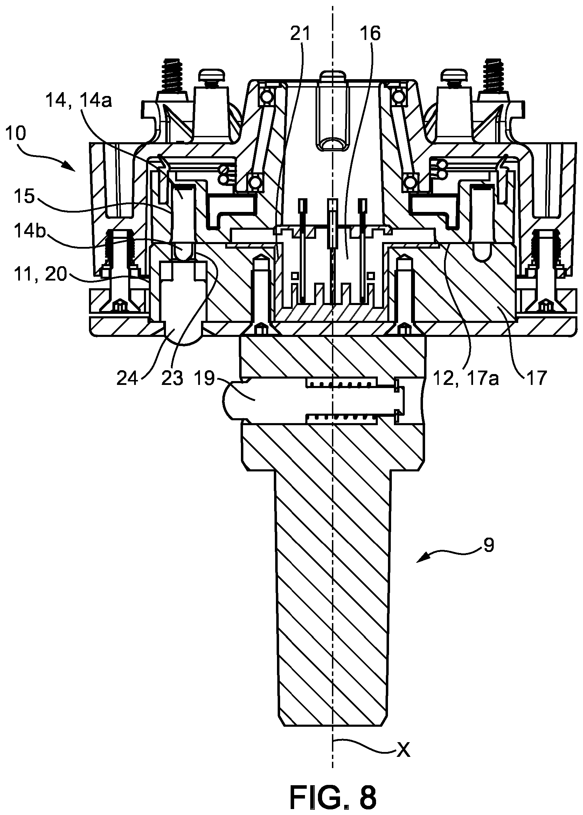

Au moins un poussoir 14 est monté dans le moyeu 10. Le poussoir 14 comporte un corps 14a (figure 8) vissé dans un trou taraudé 15 du moyeu 14 et un doigt d'indexage14b logé en partie et de façon mobile dans le corps 14a. Le doigt 14b est mobile entre une position désactivée dans laquelle elle est entièrement logée dans le corps 14a du poussoir 14 et ne fait pas saillie au-delà de la surface de fond 12, et une position activée dans laquelle le doigt 14b s'étend en partie à l'extérieur du corps 14a, en saillie par rapport à la surface de fond 12 du moyeu 10. Un ressort élastique de rappel est logé dans le corps 14a et repousse le doigt 14b vers sa position activée.

Le moyeu 10 peut également comporter des moyens de connexion électrique 16, au centre, qui peuvent être utilisés avec d'autres poignées spécifiques.

La poignée 9 comporte une embase 17 et une partie amovible non représentée, montée de façon amovible par rapport à l'embase 17. Un bouton 19 permet de libérer la partie amovible 18 par rapport à l'embase 17.

L'embase 17 comporte une surface cylindrique 20 de guidage d'axe X, apte à coopérer et à être guidée à l'intérieur de la surface de guidage cylindrique 11 du moyeu 10. L'embase 17 comporte également un évidement 21 apte à loger les moyens de connexion électrique 16 du moyeu 10.

L'embase 17 comporte en outre trois trous oblongs 22, destinés à coopérer avec les plots d'accrochage 13.

Chaque trou oblong 22 s'étend circonférentiellement autour de l'axe X, sur une plage angulaire comprise entre 5 et 25°.

On définit par largeur du trou oblong 22 la dimension du trou oblong perpendiculairement à la direction d'extension dudit trou oblong, c'est-à-dire la dimension du trou oblong selon la direction radiale.

Chaque trou oblong 22 comporte une première partie angulaire 22a et une seconde partie angulaire 22b. La largeur L1 de la première partie 22a est supérieure à la largeur L2 de la seconde partie 22b. Par ailleurs, la largeur L1 de la première partie 22a de chaque trou oblong 22 est supérieure au diamètre de la tête 13b du plot d'accrochage 13 correspondant. De plus, la largeur L2 de la seconde partie 22b de chaque trou oblong 22 est inférieure au diamètre de la tête 13b et supérieure au diamètre de la partie principale 13a du plot d'accrochage 13 correspondant.

L'embase 17 comporte en outre un logement 23 apte à recevoir au moins une partie du doigt 14b du poussoir 14.

L'embase 17 est enfin équipée d'un bouton de désactivation 24 monté dans un logement 25, ledit logement 25 débouchant dans l'orifice 23 destinée à venir en regard du doigt 14b du poussoir 14 dans une position angulaire de verrouillage de l'embase 17 par rapport au moyeu 10. Le bouton 24 est apte à être déplacé en translation le long du logement 25, une extrémité 24a du bouton 24 étant apte à pénétrer dans l'orifice 23 et à venir en appui sur le doigt 14b dans la position de verrouillage précitée.

Afin de monter l'embase 17 de la poignée 9 sur le moyeu 10 du corps, les plots d'accrochage 13 sont introduits dans les premières parties 22a des trous oblongs 22 correspondants, jusqu'à ce que les têtes 13b desdits plots d'accrochage 13 s'étendent axialement au-delà desdits trous oblongs 22, les parties principales 13a des plots d'accrochage 13 s'étendant axialement au travers des trous oblongs 22.

Par ailleurs, la surface radiale 17a dite arrière de l'embase 17 est en appui sur la surface de fond 12 du moyeu 10, le doigt 14b étant repoussé vers sa position désactivée par appui sur la surface arrière 17a de l'embase 17. Un tel effort appliqué sur l'embase 17 par le doigt 14b permet de repousser la poignée 9 et éviter qu'elle reste maintenue sur le moyeu 10 alors qu'elle n'est pas verrouillée.

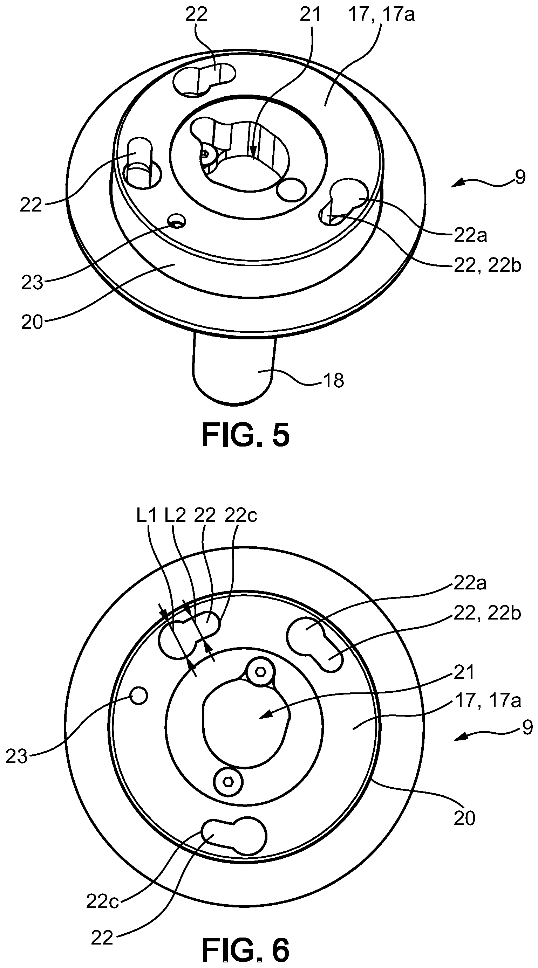

L'embase 17 est ensuite pivotée de façon à ce que les plots d'accrochage 13 se déplacent le long des trous oblongs 22 et soient situés au niveau des secondes parties 22b des trous oblongs 22. Dans cette position, les parties principales 13a sont en appui sur les extrémités circonférentielles 22c correspondantes (figure 6) des trous oblongs 22 et les têtes 13b des plots d'accrochage 13 sont en appui axial sur les bords des secondes parties 22b des trous oblongs 22, empêchant la translation de l'embase 17 par rapport au moyeu 10. Par ailleurs, le doigt 14b du poussoir 14 est repoussé vers sa position activée, en venant se loger en partie à l'intérieur du logement correspondant 23 de l'embase 17.

Le doigt 14b empêche ainsi la rotation de l'embase 17 par rapport au moyeu 10 et, ainsi, le déverrouillage de l'embase 17 par rapport au moyeu 10.

Afin de réaliser un tel déverrouillage, il est nécessaire d'appuyer sur le bouton de désactivation 24, ce dernier repoussant le doigt 14b vers sa position désactivée, à l'encontre du ressort de rappel du poussoir 14. Dès lors, l'embase 17 peut être pivotée par rapport au moyeu 10 de façon à déplacer les plots 13 vers la première partie 22a des trous oblongs 22, où le retrait de l'embase 17 par translation par rapport au moyeu 10 est possible.

## Revendications

1. Dispositif d'éclairage (1) comportant un corps (4) équipé d'au moins un module d'éclairage et d'une poignée (9) montée sur le corps (4), **caractérisé en ce que** le corps (4) comporte au moins un plot d'accrochage (13) et des moyens d'indexation (14, 23), la poignée (9) comportant au moins une embase (17) apte à pivoter autour d'un axe (X) par rapport au corps (4) entre une première position angulaire formant une position déverrouillée de l'embase (17) par rapport au corps (4) et une seconde position angulaire formant une position verrouillée de l'embase (17) sur le corps (4), ladite embase (17) comportant au moins un trou oblong (22) s'étendant circonférentiellement autour de l'axe (X) et apte à coopérer avec le plot d'accrochage (13) de façon à :
- autoriser le déplacement circonférentiel du plot d'accrochage (13) le long du trou oblong (22) correspondant entre les positions verrouillée et déverrouillée de l'embase (17) par rapport au corps (4),
- autoriser la translation selon ledit axe de l'embase (17) par rapport au corps (4) dans la position déverrouillée de l'embase (17) par rapport au corps (4), et
- interdire ladite translation dans la position verrouillée de l'embase (17) par rapport au corps (4),
les moyens d'indexation (14) du corps (4) étant aptes à coopérer avec des moyens d'indexation complémentaires (23) de l'embase (17), de façon à :
- autoriser la rotation de l'embase (17) par rapport au corps (4), de la position déverrouillée vers la position verrouillée,
- interdire la rotation de l'embase (17) par rapport au corps (4), une fois la position déverrouillée atteinte.

2. Dispositif (1) selon la revendication précédente, dans laquelle le plot d'accrochage (13) comporte une partie principale (13a) et extrémité libre (13b) de plus grande dimension que la partie principale (13a), le trou oblong (22) de la base comporte une première partie angulaire (22a) de dimension (L1) supérieure à l'extrémité libre (13b) et une seconde partie angulaire (22b) de dimension (L2) inférieure à l'extrémité libre (13b), la partie principale (13a) du plot (13) s'étendant dans la première partie angulaire (22a) du trou oblong (22) dans la position déverrouillée, la partie principale (13a) du plot (13) s'étendant dans la seconde partie angulaire (22b) du trou oblong (22) dans la position verrouillée.

3. Dispositif (1) selon l'une des revendications précédentes, dans lequel le corps (4) comporte au moins trois plots d'accrochage (13) répartis autour de l'axe de rotation de l'embase (17), coopérant respectivement avec trois trous oblongs (22) de l'embase (17).

4. Dispositif (1) selon la revendication précédente, dans lequel les plots d'accrochage (13) et les trous oblongs (22) sont répartis de façon non régulière autour de l'axe de rotation (X) de l'embase (17).

5. Dispositif (1) selon l'une des revendications précédentes, dans lequel les moyens d'indexation (14) du corps (4), ou respectivement les moyens d'indexation complémentaires de l'embase, comportent un poussoir (14) comprenant une partie mobile (14b) entre une position activée et une position désactivée, ladite partie mobile (14b) étant soumise à l'action d'un élément de rappel élastique tendant à rappeler ladite partie mobile (14b) du poussoir (14) vers sa position activée, les moyens d'indexation complémentaires (23) de l'embase (17), ou respectivement les moyens d'indexation du corps, comportant un logement (23), ladite partie mobile (14b) du poussoir (14) étant apte à être logée en partie dans ledit logement (23) de l'embase (17) en position activée.

6. Dispositif (1) selon l'une des revendications précédentes, dans laquelle le corps (4) et/ou l'embase (17) est équipée de moyens de désactivation (24) des moyens d'indexation (14, 23), aptes à libérer en rotation l'embase (17) par rapport au corps (4).

7. Dispositif (1) selon les revendications 5 et 6, dans laquelle les moyens de désactivation comportent un bouton poussoir (24) apte à repousser la partie mobile (14b) du poussoir (14) vers sa position désactivée, à l'encontre de l'effort exercé par l'élément de rappel élastique.

8. Dispositif (1) selon l'une des revendications précédentes, dans lequel l'embase (17) est apte à pivoter, autour de l'axe (X) et entre les deux positions angulaires verrouillée et déverrouillée, d'un angle compris entre 5 et 25°.

9. Dispositif (1) selon l'une des revendications précédentes dans lequel la poignée (9) comporte une partie amovible, fixée de façon amovible sur l'embase (17) par l'intermédiaire de moyens de verrouillage (19).

## Patentansprüche

1. Beleuchtungsvorrichtung (1), umfassend einen Körper (4), der mit mindestens einem Beleuchtungsmodul und einem am Körper (4) angebrachten Griff (9) ausgestattet ist, **dadurch gekennzeichnet, dass** der Körper (4) mindestens einen Befestigungszapfen (13) und Positioniermittel (14, 23) umfasst, wobei der Griff (9) mindestens eine Basis (17) umfasst, die in der Lage ist, um eine Achse (X) relativ zu dem Körper (4) zwischen einer ersten Winkelposition, die eine entriegelte Position der Basis (17) relativ zu dem Körper (4) bildet, und einer zweiten Winkelposition, die eine verriegelte Position der Basis (17) an dem Körper (4) bildet, zu schwenken, wobei die Basis (17) mindestens ein Langloch (22) umfasst, das sich in Umfangsrichtung um die Achse (X) erstreckt und in der Lage ist, mit dem Befestigungszapfen (13) zusammenzuwirken, um:
die Umfangsbewegung des Befestigungszapfens (13) entlang des entsprechenden Langlochs (22) zwischen der verriegelten und der entriegelten Position der Basis (17) relativ zum Körper (4) zu ermöglichen,
die Translation entlang der Achse der Basis (17) relativ zum Körper (4) in der entriegelten Position der Basis (17) relativ zum Körper (4) zu ermöglichen, und
die Translation in der verriegelten Position der Basis (17) relativ zum Körper (4) zu verhindern,
wobei die Positioniermittel (14) des Körpers (4) in der Lage sind, mit komplementären Positioniermitteln (23) der Basis (17) zusammenzuwirken, um:
die Drehung der Basis (17) relativ zum Körper (4) aus der entriegelten Position in die verriegelte Position zu ermöglichen,
die Drehung der Basis (17) relativ zum Körper (4) zu verhindern, sobald die entriegelte Position erreicht ist.

2. Vorrichtung (1) nach dem vorhergehenden Anspruch, wobei der Befestigungszapfen (13) einen Hauptteil (13a) und ein freies Ende (13b) mit größeren Abmessungen als der Hauptteil (13a) umfasst, wobei das Langloch (22) der Basis einen ersten Winkelteil (22a) mit einer Abmessung (L1) größer als das freie Ende (13b) und einen zweiten Winkelteil (22b) mit einer Abmessung (L2) kleiner als das freie Ende (13b) umfasst, wobei sich der Hauptteil (13a) des Zapfens (13) in der entriegelten Position in den ersten Winkelteil (22a) des Langlochs (22) erstreckt und sich der Hauptteil (13a) des Zapfens (13) in der verriegelten Position in den zweiten Winkelteil (22b) des Langlochs (22) erstreckt.

3. Vorrichtung (1) nach einem der vorhergehenden Ansprüche, wobei der Körper (4) mindestens drei Befestigungszapfen (13) umfasst, die um die Drehachse der Basis (17) verteilt sind und jeweils mit drei Langlöchern (22) der Basis (17) zusammenwirken.

4. Vorrichtung (1) nach dem vorhergehenden Anspruch, wobei die Befestigungszapfen (13) und die Langlöcher (22) ungleichmäßig um die Drehachse (X) der Basis (17) verteilt sind.

5. Vorrichtung (1) nach einem der vorhergehenden Ansprüche, wobei die Positioniermittel (14) des Körpers (4) bzw. die komplementären Positioniermittel der Basis einen Drücker (14) mit einem zwischen einer aktivierten Position und einer deaktivierten Position beweglichen Teil (14b) umfassen, wobei der bewegliche Teil (14b) der Wirkung eines elastischen Rückstellelements ausgesetzt ist, das dazu neigt, den beweglichen Teil (14b) des Drückers (14) in seine aktivierte Position zurückzuführen, wobei die komplementären Positioniermittel (23) der Basis (17) bzw. die Positioniermittel des Körpers eine Aufnahme (23) umfassen, wobei der bewegliche Teil (14b) des Drückers (14) in der Lage ist, in der aktivierten Position teilweise in der Aufnahme (23) der Basis (17) aufgenommen zu werden.

6. Vorrichtung (1) nach einem der vorhergehenden Ansprüche, wobei der Körper (4) und/oder die Basis (17) mit Deaktivierungsmittel (24) der Positioniermittel (14, 23) ausgestattet ist, die in der Lage sind, die Rotation der Basis (17) relativ zum Körper (4) freizugeben.

7. Vorrichtung (1) nach den Ansprüchen 5 und **6,** wobei die Deaktivierungsmittel einen Druckknopf (24) umfassen, der in der Lage ist, den beweglichen Teil (14b) des Drückers (14) gegen die vom elastischen Rückstellelement ausgeübte Kraft in seine deaktivierte Position zu drücken.

8. Vorrichtung (1) nach einem der vorhergehenden Ansprüche, wobei die Basis (17) in der Lage ist, um die Achse (X) und zwischen den beiden verriegelten und entriegelten Winkelpositionen um einen Winkel zwischen 5 und 25° zu schwenken.

9. Vorrichtung (1) nach einem der vorhergehenden Ansprüche, wobei der Griff (9) einen abnehmbaren Teil umfasst, der mittels Verriegelungsmitteln (19) abnehmbar an der Basis (17) befestigt ist.

## Claims

1. A lighting device (1) comprising a body (4) equipped with at least one lighting module and a handle (9) mounted on the body (4), **characterized in that** the body (4) comprises at least one attachment stud (13) and indexing means (14, 23), the handle (9) comprising at least one base (17) configured to pivot about an axis (X) relative to the body (4) between a first angular position forming an unlocked position of the base (17) relative to the body (4), and a second angular position forming a locked position of the base (17) on the body (4), said base (17) comprising at least one oblong hole (22) extending circumferentially about the axis (X) and configured to cooperate with the attachment stud (13) so as to:
- allow the circumferential movement of the attachment stud (13) along the corresponding oblong hole (22) between the locked and unlocked positions of the base (17) relative to the body (4),
- allow the translation along said axis of the base (17) relative to the body (4) in the unlocked position of the base (17) relative to the body (4), and
- prevent said translation in the locked position of the base (17) relative to the body (4),
the indexing means (14) of the body (4) being configured to cooperate with complementary indexing means (23) of the base (17), so as to:
- allow the rotation of the base (17) relative to the body (4), from the unlocked position to the locked position,
- prevent the rotation of the base (17) relative to the body (4), once the unlocked position is reached.

2. The device (1) according to the preceding claim, wherein the attachment stud (13) comprises a main portion (13a) and a free end (13b) of larger dimension than the main portion (13a), the oblong hole (22) of the base comprises a first angular portion (22a) of dimension (L1) greater than the free end (13b), and a second angular portion (22b) of dimension (L2) smaller than the free end (13b), the main portion (13a) of the stud (13) extending into the first angular portion (22a) of the oblong hole (22) in the unlocked position, the main portion (13a) of the stud (13) extending into the second angular portion (22b) of the oblong hole (22) in the locked position.

3. The device (1) according to one of the preceding claims, wherein the body (4) comprises at least three attachment studs (13) distributed about the axis of rotation of the base (17), cooperating respectively with three oblong holes (22) of the base (17).

4. The device (1) according to the preceding claim, wherein the attachment studs (13) and the oblong holes (22) are distributed irregularly about the axis of rotation (X) of the base (17).

5. The device (1) according to one of the preceding claims, wherein the indexing means (14) of the body (4), or, respectively, the complementary indexing means of the base, comprise a push button (14) that includes a movable portion (14b) between an activated position and a deactivated position, said movable portion (14b) being subjected to the action of a resilient return element tending to return said movable portion (14b) of the push button (14) to its activated position, the complementary indexing means (23) of the base (17), or, respectively, the indexing means of the body, comprising a housing (23), said movable portion (14b) of the push button (14) being configured to be partially housed in said housing (23) of the base (17) in the activated position.

6. The device (1) according to one of the preceding claims, wherein the body (4) and/or the base (17) is equipped with means (24) for deactivating the indexing means (14, 23), configured to release the base (17) in rotation relative to the body (4).

7. The device (1) according to claims 5 and 6, wherein the deactivation means comprise a push button (24) configured to push the movable portion (14b) of the push button (14) towards its deactivated position, against the force exerted by the resilient return element.

8. The device (1) according to one of the preceding claims, wherein the base (17) is configured to pivot, about the axis (X) and between the two angular positions, locked and unlocked, by an angle of between 5 and **25°.**

9. The device (1) according to one of the preceding claims, wherein the handle (9) comprises a removable portion, attached to the base (17) in a removable manner by means of locking means (19).
